# EUROPEAN PATENT APPLICATION

(11) **EP 4 289 494 A1**
(43) Date of publication of application: **13.12.2023**
(21) Application number: 22758915.7
(22) Date of filing: 24.02.2022
(51) Int. Cl.: B01D 36/00, C12M 1/00

(54) **CELL LYSIS BUFFER SEPARATION APPARATUS FOR PREPARING POLYPEPTIDE, AND SYSTEM AND APPLICATION THEREOF**

(30) Priority: 26.02.2021 CN 202120432793 U; 26.02.2021 CN 202120425267 U; 26.02.2021 CN 202110217372
(71) Applicant: Chongqing Claruvis Pharmaceutical Co., Ltd., Chongqing 400713 (CN)
(72) Inventor: ZHANG, Yan, Chongqing 400722 (CN)
(74) Representative: HGF
(86) International application number: PCT/CN2022/077629
(87) International publication number: WO 2022/179551

(57) **Abstract**

A cell lysis buffer separation apparatus for preparing a polypeptide, and a system and the application thereof. The system comprises a polypeptide enrichment module, wherein the polypeptide enrichment module comprises a cell lysis buffer separation apparatus, and the cell lysis buffer separation apparatus comprises a multi-filtration device and a waste discharge pipeline, the waste discharge pipeline being connected to the multi-filtration device. The system can perform multi-filtration treatment on a lysis buffer. Therefore, the yield of target protein can be increased; and a waste liquid obtained by means of the system contains almost no toxin polypeptide precursor with low toxicity, and has very little toxicity to an operating environment, and same can be directly discharged after simple disinfection treatment, thereby greatly reducing production cost and promoting large-scale industrial production of polypeptide under the conditions of ensuring the safety of the environment and the safety of operators.

## Description

### PRIORITY

The present application requires priority to Application No. 202120432793X, filed Feb. 26, 2021, Application No. 2021204252670, filed Feb. 26, 2021, and Application No. 202110217372X, filed Feb. 26, 2021, which are incorporated herein in their entirety by reference.

### TECHNICAL FIELD

The present invention belongs to the field of biotechnology and manufacturing, and particularly relates to a cell lysis buffer separation apparatus and system for preparing a polypeptide and application thereof.

### BACKGROUND

A polypeptide is a class of compounds formed by the connection of a plurality of amino acids through peptide bonds, and generally consists of 10-100 amino acid molecules, which is connected in the same way as a protein, with a relative molecular mass of less than 10000. Polypeptides are very common in organisms. Tens of thousands of polypeptides have been found in organisms so far, which are widely involved in regulating the functional activities of various systems, organs, tissues, and cells in the organisms, playing an important role in life activities.

Polypeptide durgs refer to polypeptides with specific therapeutic effects, acquired from chemical synthesis and gene recombination or extracted from animals and plants, and consist of a specific application of polypeptides in the field of pharmaceuticals. The polypeptide is developed rapidly and becomes a hot spot in market development at present, although the development history of the polypeptide as a drug is relatively short. The polypeptide drug is mainly used for treating major diseases related to a cancer and metabolic disorder, and drugs related to these diseases have fairly important markets all over the world. At present, 200-300 polypeptide drugs are in clinical trials, 500-600 polypeptide drugs are in preclinical trials, and more polypeptide drugs are in laboratory research stages.

With the maturation of biological recombinant technology, the mass production of recombinant polypeptide molecules, especially long-chain polypeptides, by using prokaryotes or eukaryotes as host cells is an important way to produce recombinant polypeptides. The recombinant polypeptide technology has brought about a great leap in the production of enzyme formulations and polypeptide drugs, but the price of polypeptide biological products (especially polypeptide drugs) is still high at present. The very important reasons are the complex process of synthesizing, separating and purifying the recombinant polypeptides and the high cost of the production and purification. Therefore, how to achieve efficient and large-scale expression of the recombinant polypeptides, simplify the separation process, and reduce the separation cost is an important research topic of industrial biotechnology.

Especially for the preparation of some toxin polypeptides, due to the dual requirements for protecting the environment and operators, the treatment of some large-volume substances in BL-3 environment or in large-scale isolators is required, which undoubtedly increases the production cost and is not favorable for large-scale industrial production. For example, the existing industrial botulinum toxin preparation system still needs to be in BL-3 environment to use botulinum and in large-scale isolators to perform the whole cell culture and the treatment of large-volume cell lysis buffer, the production faces huge challenges, and the preparation system still has a great risk of pollution to the environment.

### SUMMARY

In order to overcome the defects described above, the present invention provides an improved cell lysis buffer separation apparatus and system for preparing a polypeptide and application thereof. A separation protection device which is adaptive to the toxicity level of each stage is designed according to the controllable regulation of the toxicity of toxin polypeptides in the production process. In one aspect, the production cost is greatly reduced. In another aspect, the strict protection on the environment is also realized. The present invention is specifically as follows.

In a first aspect of the present invention, provided is a cell lysis buffer separation apparatus, wherein the apparatus comprises a filtration device and a waste discharge pipeline, and the filtration device is a multiplex filtration device.

Preferably, the multiplex filtration device comprises a crude liquid filtration device and a feed liquid filtration flow path, and the crude liquid filtration device and the feed liquid filtration flow path are connected via a pipeline,
wherein the crude liquid refers to the cell lysis buffer before filtration, and the feed liquid refers to the substance after the crude liquid is filtered by the crude liquid filtration device.

Preferably, the cell lysis buffer is filtered by the crude liquid filtration device, and a substance that can pass through the crude liquid filtration device enters the feed liquid filtration flow path as a feed liquid.

More preferably, the feed liquid filtration flow path comprises one or more feed liquid filtration devices.

More preferably, each of the feed liquid filtration flow paths is connected to the waste discharge pipeline.

Preferably, a pipeline valve is arranged on the pipeline connecting the crude liquid filtration device and the feed liquid filtration flow path.

Preferably, the feed liquid filtration device can be recycled, and the feed liquid filtration device that can be recycled is a feed liquid circulation filtration device.

Further preferably, the feed liquid filtration flow path comprises a feed liquid circulation storage device and a feed liquid circulation filtration device, and the feed liquid circulation storage device and the feed liquid circulation filtration device are connected end to end via a pipeline to form a feed liquid circulation filtration flow path.

Preferably, a pump is further arranged on the pipeline between the tail end of the feed liquid circulation storage device and the front end of the feed liquid circulation filtration device.

Further preferably, the waste residue discharge pipeline and the waste feed liquid discharge flow path are connected to the same or different waste receiving apparatuses.

More preferably, the waste residue discharge pipeline is arranged on the crude liquid filtration flow path, and a substance that cannot pass through the crude liquid filtration flow path enters the waste residue discharge pipeline as a waste residue.

Preferably, the multiplex filtration device comprises a plurality of layers of filtration devices having different filtration pore sizes.

More preferably, the filtration material of the crude liquid filtration device has a pore size suitable for separation of a solid and liquid in the cell lysis buffer.

Preferably, the filtration material of the multiplex filtration device is selected from hydrophilic materials or hydrophobic materials. More preferably, the hydrophilic materials are selected from cellulose ester, polyethersulfone, polyethylene and the like or derivatives thereof, and the hydrophobic materials are selected from polyvinylidene fluoride, polypropylene, polytetrafluoroethylene and the like or derivatives thereof.

Preferably, the material is a filter membrane.

Preferably, the filtration material of the crude liquid filtration device on the crude liquid filtration flow path has a pore size suitable for separation of a solid and liquid in the cell lysis buffer.

Preferably, the filtration material of the crude liquid filtration device has a pore size of 0.1-0.65 µm.

More preferably, the filtration material of the feed liquid circulation filtration device has a pore size of 0.2 µm or less.

Preferably, the waste discharge pipeline comprises a waste residue discharge pipeline and a waste feed liquid discharge pipeline.

More preferably, the waste residue discharge pipeline is arranged downstream of the crude liquid filtration device, and the waste feed liquid discharge pipeline is arranged downstream of the feed liquid filtration device.

The waste residue discharge pipeline and the waste feed liquid discharge pipeline are connected to the same or different waste receiving apparatuses.

Preferably, the waste feed liquid discharge flow path is arranged on the feed liquid circulation filtration flow path.

Preferably, a pipeline valve is arranged between the waste feed liquid discharge flow path and the feed liquid circulation filtration flow path.

Preferably, the waste feed liquid discharge flow path is arranged on the feed liquid circulation storage device.

Preferably, the waste feed liquid discharge flow path is arranged on the tail end of the feed liquid circulation storage device.

Preferably, the feed liquid which still cannot reach the clarity degree of the finished liquid after the circulation filtration enters the waste feed liquid discharge flow path as a waste feed liquid.

Preferably, the feed liquid, after a certain times of circulation filtration, enters the waste feed liquid discharge flow path as a waste feed liquid.

Preferably, the apparatus further comprises a lysis buffer inlet pipeline and a supplementary liquid inlet pipeline, and the lysis buffer inlet pipeline and the supplementary liquid inlet pipeline are connected to the front end of the crude liquid filtration device.

Preferably, a pump is further arranged on the lysis buffer inlet flow path and the supplementary liquid inlet flow path.

Preferably, the supplementary liquid is a buffer.

Preferably, when the passing rate of the crude liquid filtration device is reduced to a set value, the lysis buffer stops entering the crude liquid filtration device, the supplementary liquid enters the crude liquid filtration device, and the supplementary liquid enables waste residues deposited in the crude liquid filtration device to be separated from the crude liquid filtration device and enter the waste residue discharge pipeline.

Preferably, the supplementary liquid enters the crude liquid filtration device according to a set time interval or time point.

Preferably, the supplementary liquid enters the crude liquid filtration device according to a set program.

More preferably, the lysis buffer inlet pipeline and buffer inlet pipeline may be the same or different.

Preferably, the apparatus further comprises a finished liquid collection flow path, and the finished liquid collection flow path comprises a finished liquid collection device and a real-time turbidity monitoring device .

Preferably, the device further comprises a finished liquid collection flow path.

Preferably, the finished liquid collection flow path is arranged on the feed liquid filtration flow path.

Preferably, the finished liquid collection flow path is arranged on the feed liquid circulation filtration device.

Preferably, the real-time turbidity monitoring device controls a pipeline valve on the finished liquid collection flow path.

Preferably, the feed liquid reaching the threshold set by the real-time turbidity monitoring device in the feed liquid circulation filtration enters the finished liquid collection flow path.

More preferably, the apparatus is completely enclosed.

Preferably, the apparatus can be used for the clarification of any liquid, especially liquids containing slightly toxic substances.

The apparatus provided by the present invention can ensure that the separation process of the cell lysis buffer is carried out in a completely enclosed system, thereby significantly reducing the probability of exposing the substances treated in the process steps to the environment and operators and making it particularly suitable for the separation treatment of the cell lysis buffer containing a toxic substance.

In a second aspect of the present invention, provided is a system for preparing a polypeptide, wherein the system comprises an enrichment module, the enrichment module comprises a cell lysis buffer separation apparatus, and the cell lysis buffer separation apparatus is the cell lysis buffer separation apparatus as described above.

Preferably, the system further comprises a fermentation module, and the fermentation module is located upstream of the enrichment module.

Preferably, the fermentation module comprises a host cell culture device and a lysis device, and the lysis device is arranged downstream of the culture device.

Preferably, the fermentation module further comprises a sealed storage device for host cell fermentation broth, and the sealed storage device is arranged between the culture device and the lysis device.

Preferably, the fermentation module further comprises a homogenizing device, and the homogenizing device is arranged downstream of the lysis device.

Preferably, a buffer supply device is further arranged on the sealed storage device.

Preferably, a waste advancing and retreating device is further arranged on the lysis device.

Preferably, the system further comprises a purification module, and the purification module is located downstream of the enrichment module.

Preferably, the purification module comprises at least 1 chromatography device set.

Preferably, the chromatography device comprises 2 or 3 chromatography device sets.

Preferably, the chromatography devices are the same or different; more preferably, the chromatography device is an affinity chromatography device, gel filtration chromatography device, and/or ion chromatography device.

Preferably, the chromatography device is located in an isolator.

Preferably, the purification module further comprises a protease liquid supply device.

Preferably, the protease liquid supply device is connected to the front end of the first chromatography device.

Preferably, the protease liquid supply device comprises two or more protease liquid supply devices, and the protease liquid comprises different proteases.

Preferably, the purification module comprises a finished product collection device, and the finished product collection device is arranged at the tail end of the last chromatography device.

Preferably, the purification module further comprises a cleaning liquid supply device arranged upstream of each of the chromatography devices respectively.

Preferably, the purification module further comprises an equilibration buffer supply device arranged upstream of each of the chromatography devices.

Preferably, the equilibration buffer supply device is arranged upstream of the second and third chromatography devices.

Preferably, the purification module further comprises a waste liquid collection device arranged below each of the chromatography devices respectively.

Preferably, the polypeptide is present in the form of a low-toxic single-chain polypeptide in a cell or lysis buffer and has high toxicity after a downstream protease (also called an activating enzyme) activation step.

Further preferably, the low toxicity is relative to the high toxicity after the activation. In a specific embodiment, the activity of the toxin polypeptide after the activation is at least 5000 folds higher than that of the single-chain polypeptide, and still further, the activity of the toxin polypeptide after the activation is 6000, 7000, 8000, 9000, 10000, 12000, 15000 or more folds higher than that of the single-chain polypeptide.

Preferably, the polypeptide is a gene recombinant polypeptide.

Preferably, the single-chain polypeptide is a toxin polypeptide precursor.

In a specific embodiment, the toxin polypeptide precursor is a neurotoxin precursor and has neurotoxicity after being activated by a protease, namely an activating enzyme.

In a third aspect of the present invention, provided is use of the cell lysis buffer separation apparatus or system described above in preparing a polypeptide.

Preferably, the polypeptide is present in the form of a low-toxic single-chain polypeptide in a cell or lysis buffer and has toxicity after a downstream protease activation step; preferably, the polypeptide is a gene recombinant polypeptide.

Preferably, the single-chain polypeptide is a toxin polypeptide precursor.

In a specific embodiment, the toxin polypeptide precursor is a neurotoxin precursor and has neurotoxicity after being activated by a protease, namely an activating enzyme.

Preferably, the polypeptide comprises at least two functional amino acid structural regions, wherein a first functional amino acid structural region comprises a metal ion-dependent protease activity domain, a second functional amino acid structural region comprises a receptor-binding domain that can bind to a surface receptor of a target cell and/or a translocation domain that can mediate the transfer of the polypeptide across the vesicle membrane, and the two functional amino acid structural regions are connected via a structural region comprising a protease cleavage site.

Preferably, the functional amino acid structural regions are from or are derived from the same or different natural polypeptides.

Preferably, the functional amino acid structural region is from a computer design.

Preferably, a structural region comprising the two functional amino acid structural regions described above and comprising a protease cleavage site (the second protease cleavage site) is defined as a second polypeptide fragment.

Preferably, the metal ion-dependent protease activity domain is a Zn²⁺-dependent protease activity domain.

Preferably, the first functional amino acid structural region and/or the second functional amino acid structural region are/is encoded by a natural sequence and/or an artificially synthesized sequence. More preferably, the first functional amino acid structural region of the polypeptide comprises a Zn²⁺ protease-binding domain of the light chain of clostridial neurotoxin.

Preferably, a human cell of the receptor-binding domain capable of binding to an antigen on the surface of a human cell in the second functional amino acid structural region refers to a human cell with an SNARE complex. For example: a human nerve cell, a pancreatic cell, or other cells with an SNARE complex.

More preferably, the human cell of the receptor-binding domain capable of binding to an antigen on the surface of the human cell in the second functional amino acid structural region refers to a human nerve cell or a pancreatic cell, and the receptor-binding domain is a receptor-binding domain capable of specifically binding to the human nerve cell or the pancreatic cell.

More preferably, the receptor-binding domain of the second functional amino acid structural region is a cell surface receptor-binding domain of the heavy chain of clostridial neurotoxin, and the translocation domain of the second functional amino acid structural region that mediates the transfer of the polypeptide across the membrane is a domain that mediates the transfer of clostridial neurotoxin across the membrane.

More preferably, the clostridial neurotoxin is a botulinum neurotoxin or tetanus toxin.

More preferably, the botulinum neurotoxin is selected from any one of serotypes BoNT/A-BoNT/H and derivatives thereof known in the art.

More preferably, the clostridial neurotoxin is selected from any one of tetanus toxin or a derivative thereof. More preferably, the first functional amino acid structural region comprises part or all of the light chains of BoNT/A, BoNT/B, BoNT/C, BoNT/D, BoNT/E, BoNT/F, BoNT/G, BoNT/H or tetanus toxin.

More preferably, the second functional amino acid structural region comprises part or all of the heavy chain of BoNT/A, BoNT/B, BoNT/C, BoNT/D, BoNT/E, BoNT/F, BoNT/G, BoNT/H or the tetanus toxin.

In one specific embodiment, the first functional amino acid structural region is the light chain of BoNT/A, and the second functional amino acid structural region is the heavy chain of BoNT/A.

More preferably, the first functional amino acid structural region and the second functional amino acid structural region may be from different serotypes. The first functional amino acid structural region and the second functional amino acid structural region may be any combination of the above serotypes. For example, the first functional amino acid structural region is derived from the light chain of BoNT/A and the second functional amino acid structural region is derived from the heavy chain of BoNT/B, or the first functional amino acid structural region is derived from the light chain of BoNT/A and the second functional amino acid structural region is derived from the heavy chain of BoNT/C, and the like.

Preferably, the polypeptide further comprises a first polypeptide fragment comprising a tag protein.

More preferably, the tag protein is selected from His, a glutathione S-transferase (GSTs), C-myc, chitin-binding domain, maltose-binding protein (MBP), SUMO heterologous affinity moiety, monoclonal antibody or protein A, streptavidin-binding protein (SBP), cellulose-binding domain, calmodulin-binding peptide, S tag, Strep tag II, FLA, protein A, protein G, histidine affinity tag (HAT) and polyhistidine.

In a specific embodiment, the tag protein is located at an N terminus of the single-chain polypeptide.

In a specific embodiment, the tag protein is a glutathione S-transferase (GSTs).

Preferably, the first polypeptide fragment further comprises a structural region of a first protease cleavage site.

Preferably, neither the first protease cleavage site nor the second protease cleavage site can be cleaved by a human protease or a protease produced by the host cell expressing the single-chain polypeptide.

More preferably, the first protease cleavage site and the second protease cleavage site are identical or different.

More preferably, the specific protease is selected from one of or a combination of two of a non-human enterokinase, a tobacco etch virus protease, a protease derived from Bacillus subtilis, a protease derived from Bacillus amyloliquefaciens, a protease derived from rhinovirus, papain, a homologue of papain of an insect, or a homologue of papain of a crustacean.

In a specific embodiment, the protease that specifically recognizes the first protease and the protease that specifically recognizes the second protease cleavage site are both derived from the proteases of rhinoviruses.

Preferably, the second enzyme cleavage site is embedded into, or partially replaces or completely replaces an activation loop between a first functional peptide fragment and a second functional peptide fragment. The embedding refers to an embedding between two certain amino acids in the activation loop; the partial replacement refers to that the second enzyme cleavage site replaces part of the amino acid sequence of the activation loop; the complete replacement refers to that the amino acid sequence of the natural activation loop is completely replaced by the second enzyme cleavage site.

More preferably, the first protease cleavage site and the second protease cleavage site are selected from one of or a combination of two of DDDDK, EXXYXQS/G, HY, YH, or LEVLFQGP.

In a specific embodiment, the first protease cleavage site and the second protease cleavage site are both LEVLFQGP.

Preferably, the first polypeptide fragment further comprises a short linker peptide.

Preferably, the short linker peptide enables the first protease cleavage site to be more easily recognized or bound to by a protease thereof.

More preferably, the short linker peptide does not affect the function of the single-chain polypeptide.

More preferably, the short linker peptide retained at the N terminus of the second polypeptide fragment does not affect the function of the second polypeptide fragment after the cleavage of the first protease cleavage site.

More preferably, the short linker peptide has no more than 5 amino acid residues; more preferably, the short linker peptide is selected from a glycine-serine (GS) short peptide, GGS, GGGS, GGGGS, GSGS, GGSGS, GSGGS, GGSGS, GGGSS, and other short linker peptides.

In a specific embodiment, the combination of the structural region comprising the first protease cleavage site and the short linker peptide is LEVLFQGPLGS.

In a specific embodiment, the polypeptide sequentially comprises, from the N terminus: the glutathione S-transferase, LEVLFQGPLGS, the light chain of BoNT/A, LEVLFQGP, and the heavy chain of BoNT/A. More preferably, the polypeptide has a sequence set forth in SEQ ID NO: 1.

The polypeptide, after being cleaved by the first protease to remove the tag protein, becomes a polypeptide derivative without the tag protein, and if the second protease cleavage site is recognized and cleaved by a specific protease, a toxin polypeptide with a dimer structure formed by connecting a light chain and a heavy chain via a disulfide bond is formed. The toxin polypeptide has neurotoxicity similar to a natural toxin molecule, the molecule before the peptide fragment of the light chain and the heavy chain of the single-chain polypeptide is cleaved is a precursor of the toxin polypeptide, and the precursor molecule, the polypeptide or the polypeptide derivative, has no toxicity or only slight toxicity compared with a natural molecule.

The term "polypeptide" involved in the present application refers to a natural or gene recombinant polypeptide, which is preferably from a natural or gene recombinant toxin polypeptide precursor.

The toxicity of the polypeptide is the activity of the polypeptide.

The "activation" refers to that a protease, also called an activating enzyme, is contacted with the low-toxic toxin polypeptide precursor, the peptide bond between the functional amino acid structural regions is cleaved, and the functional amino acid structural regions are connected via a disulfide bond and folded into the toxic polypeptide with physiological activity and spatial conformation.

The term "functional amino acid structural region" can be understood as a peptide chain well known to those skilled in the art, such as a light chain, a heavy chain, or a subunit, etc.

The "tag protein", which is a term understood by those skilled in the art, is a heterologous affinity reagent.

The term "completely enclosed" involved in the present application has two meanings. In one aspect, the blocking function included in the apparatus pipeline, such as a rubber gasket, a bolt, a stainless steel interface and the like, ensures that the cell lysis buffer processing apparatus does not leak in operation. In another aspect, the design principle of the cell lysis buffer processing apparatus ensures that the apparatus does not need to be disconnected to carry out manual operations such as liquid changing and the like in the working process.

The "isolator" refers to a apparatus that is conventional in the art.

The system of the present invention designs the multiplex filtration cell lysis buffer separation apparatus according to the low toxicity of the polypeptide, and thus can greatly reduce the pollution of tiny liquid particles entering the surrounding environment compared with the traditional centrifugal process. The system can adopt a plurality of different filtration pore sizes for the first filtration treatment, and the cell lysis buffer enters the circulation feed liquid storage device after the first filtration treatment is finished, and enters the second filtration treatment via pressure. For the common problems of filter membrane blockage, the need to perform liquid changing and flushing and the like in the treatment of a cell lysis buffer, the apparatus described above can improve the yield of the target protein in one aspect. In another aspect, the waste liquid obtained by the system almost contains no low-toxic toxin polypeptide precursors, with very little toxicity to the operating environment, and can be directly discharged after simple disinfection treatment. Preferably, the cell lysis buffer separation apparatus of the present invention is performed according to the following method in a completely enclosed manner: in one aspect, when visible deposition occurs in the crude liquid filtration device and the passing rate is significantly reduced, the liquid in the liquid inlet pipeline is switched from the cell lysis buffer to the supplementary liquid, and the supplementary liquid enables the deposition in the crude liquid filtration device to be separated from the crude liquid filtration device and enter a waste residue discharge pipeline connected with the crude liquid filtration device; in another aspect, when the turbidity of the feed liquid reaches a certain degree, the valve on a branch of the pipeline leading to the waste feed liquid discharge pipeline at the tail end of the feed liquid circulation storage device is opened, and the feed liquid is discharged as a waste liquid. The apparatus can prevent the leakage of toxic protein from the crude purification of the protein, and the *Escherichia coli* stock lysate only needs to be produced in a completely closed pipeline without using an isolator. The filtration and purification apparatus combined with low toxicity of the polypeptide can avoid using an isolator to carry out safe treatment and preliminary purification on a large amount of a toxic protein solution, thereby greatly reducing the production cost under the condition of ensuring safety, while the purity of the polypeptide is further improved via a plurality of chromatography steps, and the purity can reach more than 90%. The improvement described above of the system of the present invention enables a large-scale commercial production of the toxic polypeptide through recombinant protein to be possible.

### BRIEF DESCRIPTION OF THE DRAWINGS

FIG. 1 illustrates a schematic structure of a system for preparing a polypeptide of the present invention.
FIG. 2 illustrates SDS-PAGE results of GSTs-BoNT/A (lane 1) and 150 kD BoNT/A after a GSTs tag had been removed (lane 2) obtained using the system of the present invention.
FIG. 3 illustrates the SDS-PAGE results of a high-purity BoNT/A protein obtained by further purifying the product without GSTs tag through an ion exchange column.

### DETAILED DESCRIPTION

The present invention is further described with reference to the following specific examples, and the advantages and features of the present invention will be clearer as the description proceeds. These examples are illustrative only and do not limit the scope of the present invention in any way. It should be appreciated by those skilled in the art that modifications and replacements can be made to the details and form of the technical solutions of the present invention without departing from the spirit and scope of the present invention and that all these modifications and replacements fall within the scope of the present invention.

### Example 1: System for Preparing Polypeptide

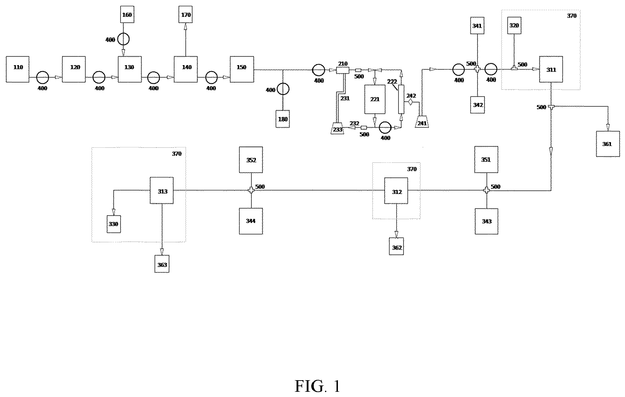

As shown in FIG. 1, a system for preparing a polypeptide sequentially comprises:
(1) A fermentation module, sequentially comprising a constant temperature shaker 110, a fermentation device 120, a sealed storage tank 130, a lysis device 140, and a homogenizing device 150, wherein a buffer supply device 160 is further arranged on the sealed storage tank 130, and a waste advancing and retreating device 170 is further arranged on the lysis device 140; the above devices are connected via a pipeline, a pump 400 is arranged on the pipeline, and the pump 400 is respectively arranged between the constant temperature shaker 110 and the fermentation device 120, between the fermentation device 120 and the sealed storage tank 130, between the sealed storage tank 130 and the lysis device 140, between the lysis device 140 and the homogenizing device 150, and between the sealed storage tank 130 and the buffer supply device 160.
(2) An enrichment module. Herein, the enrichment module refers to a cell lysis buffer processing apparatus and comprises a crude liquid filtration device 210, a feed liquid circulation storage device 221, a feed liquid circulation filtration device 222, a waste residue discharge pipeline 231, a waste liquid discharge pipeline 232, a waste collection device 233, a finished liquid collection device 241, a real-time turbidity monitoring and valve device 242, and a buffer supply device 180. All the devices are connected via a pipeline, wherein the front end of the crude liquid filtration device 210 is connected with the upstream fermentation module and buffer supply device 180, the crude liquid filtration device 210 is connected with the waste residue discharge pipeline 231, and the tail end of the crude liquid filtration device 210 is connected with a feed liquid filtration device formed by connecting the feed liquid circulation storage device 221 and the feed liquid circulation filtration device 222 end to end via a pipeline; the tail end of the feed liquid circulation storage device 221 is also connected with the waste liquid discharge pipeline 232, the feed liquid circulation filtration device 222 is also connected with a finished liquid collection flow path, and the finished liquid collection flow path is composed of the real-time turbidity monitoring and valve device 242 and the finished liquid collection apparatus 241. Pumps are arranged on the pipeline at the front end of the crude liquid filtration device 210 and the pipeline between the tail end of the feed liquid circulation storage device 221 and the front end of the feed liquid circulation filtration device 222. A pipeline valve is arranged on the pipeline leading from the tail end of the feed liquid circulation storage device 221 to the waste liquid discharge pipeline 232. All the devices are connected via a pipeline. The finished liquid collection device 241 is connected with a downstream purification module. The crude liquid filtration device has a pore size of 0. 1-0.65 µm, the filtration material of the feed liquid circulation filtration device has a pore size of 0.2 µm or less, and the material may be any hydrophilic or hydrophobic filtering material.
(3) A purification module, sequentially comprising a chromatography device I 311, a chromatography device II 312, and a chromatography device III 313, wherein an activating enzyme supply device 320 is arranged at the front end of the chromatography device 1311, and a finished product collection device 330 is arranged at the tail end of the chromatography device III 313; a cleaning liquid supply device 340 and a chromatography equilibration buffer supply device 350 are arranged at the front end of each of the chromatography devices, and a waste liquid collection device 360 is arranged at the tail end of each of the chromatography devices; an isolator 370 is arranged outside each of the chromatography devices. The protease liquid supply device may further comprise two or more protease liquid supply devices, and the protease liquid comprises different proteases. (1), (2), and (3) are connected via a pipeline.

### Example 2: Expression of GSTs-BoNT/A

A host cell expressing the target polypeptide was prepared using genetic engineering technology (Molecular Cloning A Laboratory Manual, 2nd Ed., ed. By Sambrook, Fritsch and Maniatis. Cold Spring Harbor Laboratory Press: 1989), which comprises: 1. designing and constructing a nucleic acid molecule encoding a polypeptide of a modified neurotoxin; 2. constructing a plasmid containing the nucleic acid molecule in step 1; 3. transfecting the plasmid constructed in step 2 into a host cell; 4. inoculating, culturing, fermenting, lysing and homogenizing *Escherichia coli* expressing the polypeptide using the fermentation module in the system of the present invention.

The amino acid sequence of the expressed polypeptide is set forth in SEQ ID NO: l.

### Example 3: Enrichment of GSTs-BoNT/A

The crude cell lysis buffer obtained in Example 2 was enriched using the system in Example 1: the lysate entered the completely enclosed enrichment module via a pump, namely a completely enclosed cell lysis buffer separation and purification instrument. In the completely enclosed cell lysis buffer separation and purification instrument, firstly, a first layer of the crude liquid filtration device was used for separating the lysis buffer and residue, the residue entered a waste recycling device, and the filtered lysis buffer further entered the feed liquid circulation filtration device as a feed liquid. In the circulation filtration process, the circulation liquid meeting the finished liquid collection condition entered the finished liquid collection device, and the circulation liquid not meeting the finished liquid collection condition continued to be subjected to the circulation filtration or entered a waste liquid recycling device.

Aiming at the problems of filter membrane blockage, the need to perform liquid changing and flushing and the like in the treatment of a cell lysis buffer, in the cell lysis buffer device of the present application, in one aspect, when visible deposition occurred in the crude liquid filtration device and the passing rate was significantly reduced, the liquid in the liquid inlet pipeline was switched from the cell lysis buffer to buffer II, and the buffer II enabled the deposition in the crude liquid filtration device to be separated from the crude liquid filtration device and enter a waste residue discharge pipeline connected with the crude liquid filtration device; in another aspect, when the turbidity of the feed liquid reached a certain degree, the valve on a branch of the pipeline leading to the waste feed liquid discharge pipeline at the tail end of the feed liquid circulation storage device was opened, and the feed liquid was discharged as a waste liquid. The whole process did not need manual operations such as liquid changing, flushing and the like.

The finished liquid collection condition was that at least two circulations were performed.

By the completely enclosed cell lysis buffer separation apparatus, the obtained finished liquid contained more than 90% of polypeptide, and the obtained waste liquid almost contained no single-chain polypeptide, with very little toxicity to the operating environment, and could be directly discharged after simple disinfection treatment.

### Example 4: Cleavage of GSTs Tag Protein and Purification of BoNT/A

The product in Example 2 entered the purification module via the enrichment module and was contacted with a GSTs affinity ligand in a first chromatography device (an affinity chromatography device). Substances which were not bound to the ligand were eluted by a conventional method, and the GSTs were dissociated from the ligand by a conventional method to obtain the preliminarily purified GSTs-BoNT/A.

The conventional method is as follows: a chromatographic column was washed with 20 column volumes of a phosphate buffer. GSTs-BoNT/A elution was carried out with 10 column volumes of a freshly prepared 10 mM glutathione eluent buffer (0.154 g of reduced glutathione dissolved in 50 mL of 50 mM Tris-HCl (pH 8.0)). The elution of the fusion protein was monitored by absorbance reading at 280 nm.

After elution, GSTs tag protein was cleaved under the effect of Rinovirus 3C Protease.

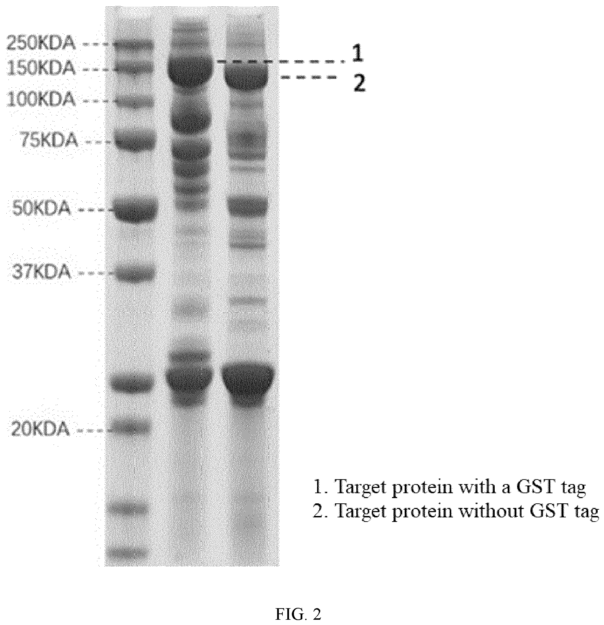

As shown in FIG. 2, compared with lane 1 in which the tag protein was not cleaved, the GSTs tag protein of lane 1 was cleaved to obtain a BoNT/A molecule without GSTs under the effect of Rinovirus 3C Protease.

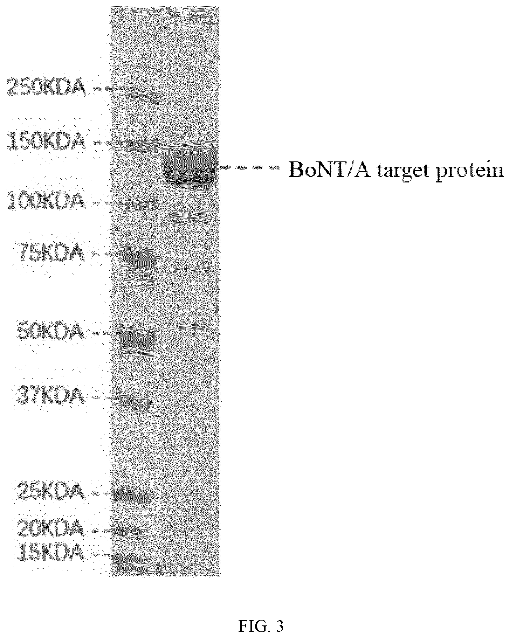

The preliminarily purified product was further purified in a second chromatography device (a gel filtration chromatography device), or a third chromatography device (an ion chromatography device). The purification method was well known to those skilled in the art. The results are shown in FIG. 3. The band corresponding to the polypeptide is significantly thickened, and a BoNT/A molecule with the purity of 90% can be obtained.

### Example 5: Toxicity Test of GSTs-BoNT/A and BoNT/A

The GSTs-BoNT/A obtained in Example 4 had an LD₅₀ of 45-450 ng after intraperitoneal injection in mice; considering the purity of the injected botulinum toxin protein, the converted LD₅₀ was 22.5-225 ng, with a mid-value of 123.75 ng. The BoNT/A obtained in Example 4 had an LD₅₀ of 0.02-0.05 ng after intraperitoneal injection in mice. Considering the purity of the injected botulinum toxin protein, the converted LD₅₀ was 0.006-0.015 ng, with a mid-value of 0.0105 ng (See Table 1).

**Table 1. Toxicity comparison of GSTs-BoNT/A and BoNT/A molecules in mice biotoxicity study**

| **Test sample** | **Description** | **Dose (ng)** | **Lethality rate of mice 72 h after administration (%)** |
|---|---|---|---|
| GST-BoNT/A (50% purity) | GST-BoNT/A (50% purity) | 450 | 100 |
| | | 45 | 50 |
| | | 4.5 | 0 |
| | | 0.45 | 0 |
| BoNT/A (30% purity) | BoNT/A (30% purity) | 0.05 | 100 |
| | | 0.02 | 0 |
| | | 0.01 | 0 |
| | | 0.005 | 0 |

The GSTs-BoNT/A had the activity of botulinum toxin, and the median lethal dose (LD₅₀) thereof was approximately 11786 times higher than the LD₅₀ of the BoNT/A protein after intraperitoneal injection in mice, which indicates that the activity of the toxin precursor molecule of GSTs-BoNT/A recombinant protein is approximately 11786 times weaker than that of the final product BoNT/A. The experiment demonstrates that the toxin precursor molecule of the GSTs-BoNT/A recombinant protein has the activity of botulinum toxin, but toxicity much lower than that of the activated BoNT/A. Therefore, the cell lysis buffer separation apparatus in which the cell lysis buffer contains the the toxin polypeptide precursor is subjected to a completely enclosed treatment, namely the blocking function included in the apparatus pipeline, such as a rubber gasket, a bolt, and a stainless steel interface, and in the operation process, the apparatus does not need to be disconnected to carry out manual operations such as flushing, liquid changing and the like. The completely enclosed cell lysis buffer processing apparatus can completely process the cell lysis buffer containing the low-toxic toxin polypeptide precursor without an additional enclosed working space, such as an isolator.

The examples of the present application demonstrate that the system for preparing a polypeptide claimed in the present application can be applied to the preparation of a toxic polypeptide, which is activated as a toxin molecule with natural toxicity only by hydrolysis with a specific protease, and is present in a low-toxic form in the host cell and cell lysis buffer. In this case, cell fermentation, lysis, and lysis buffer separation can all be performed in the enclosed system of the present application without the need for an isolator in these steps, which in one aspect improves the safety of the production of low-toxic polypeptides and in another aspect reduces the production cost. Meanwhile, the multi-step chromatography purification after the protease activation further improves the purity of the active polypeptide, and the purity can reach 90%, thereby enabling the large-scale industrial production of such polypeptides to be possible.

The preferred embodiments of the present invention are described in detail above, which, however, are not intended to limit the present invention. Within the scope of the technical concept of the present invention, various simple modifications can be made to the technical solution of the present invention, all of which will fall within the protection scope of the present invention.

In addition, it should be noted that the various specific technical features described in the above specific embodiments can be combined in any suitable manner where the features do not contradict each other. In order to avoid unnecessary repetition, such combinations will not be illustrated separately.

## Claims

1. A cell lysis buffer separation apparatus, wherein the apparatus comprises a filtration device and a waste discharge pipeline, the filtration device is a multiplex filtration device.

2. The apparatus according to claim 1, wherein the multiplex filtration device comprises a crude liquid filtration device and a feed liquid filtration flow path, and the crude liquid filtration device and the feed liquid filtration flow path are connected via a pipeline; preferably, the feed liquid filtration flow path comprises one or more feed liquid filtration devices; more preferably, the feed liquid filtration flow path comprises a feed liquid circulation storage device and a feed liquid circulation filtration device, the feed liquid circulation storage device and the feed liquid circulation filtration device are connected end to end via a pipeline to form a feed liquid circulation filtration flow path.

3. The apparatus according to any one of claims 1-2, wherein the multiplex filtration device comprises a plurality of layers of filtration devices having different filtration pore sizes; preferably, the filtration material of the crude liquid filtration device has a pore size suitable for separation of a solid and liquid in the cell lysis buffer.

4. The apparatus according to claim 3, wherein the filtration material of the multiplex filtration device is selected from hydrophilic materials or hydrophobic materials, the hydrophilic materials are selected from cellulose ester, polyethersulfone, polyethylene and the like or derivatives thereof, and the hydrophobic materials are selected from polyvinylidene fluoride, polypropylene, polytetrafluoroethylene and the like or derivatives thereof.

5. The apparatus according to any one of claims 2-4, wherein the filtration material of the crude liquid filtration device has a pore size of 0.1-0.65 µm, and the filtration material of the feed liquid circulation filtration device has a pore size of 0.2 µm or less.

6. The apparatus according to any one of claims 1-5, wherein the waste discharge pipeline comprises a waste residue discharge pipeline and a waste feed liquid discharge pipeline; preferably, the waste residue discharge pipeline is arranged downstream of the crude liquid filtration device, the waste feed liquid discharge pipeline is arranged downstream of the feed liquid filtration device.

7. The apparatus according to any one of claims 1-6, wherein the apparatus further comprises a lysis buffer inlet pipeline and a supplementary liquid inlet pipeline, and the lysis buffer inlet pipeline and the supplementary liquid inlet pipeline are connected to the front end of the crude liquid filtration device; preferably, the lysis bufferinlet pipeline and buffer inlet pipeline may be the same or different.

8. The apparatus according to any one of claims 1-7, wherein the apparatus further comprises a finished liquid collection flow path, the finished liquid collection flow path comprises a finished liquid collection device and a real-time turbidity monitoring device.

9. The apparatus according to any one of claims 1-8, wherein the apparatus is completely enclosed.

10. A system for preparing a polypeptide, wherein the system comprises an enrichment module, the enrichment module comprises a cell lysis buffer separation apparatus, and the cell lysis buffer separation apparatus is the cell lysis buffer separation apparatus according to any one of claims 1-9.

11. The system according to claim 10, wherein the system further comprises a fermentation module upstream of the enrichment module; preferably, the fermentation module comprises a culture, fermentation, and lysis device of the host cell.

12. The system according to any one of claims 10-11, wherein the system further comprises a purification module downstream of the enrichment module.

13. The system according to claim 12, wherein the purification module comprises at least 1 chromatography device set; preferably, the purification module comprises 3 chromatography device sets; preferably, the chromatography device is located in an isolator; preferably, the purification module further comprises a protease liquid supply device.

14. The system according to any one of claims 10-13, wherein the polypeptide is present in the form of a low-toxic single-chain polypeptide in a cell or lysis bufferand has toxicity after a downstream protease activation step; preferably, the polypeptide is a gene recombinant polypeptide.

15. The system according to claim 14, wherein the polypeptide is a neurotoxin precursor and has neurotoxicity after being activated by a protease.

16. Use of the cell lysis buffer separation apparatus according to any one of claims 1-9 or the system for preparing a polypeptide according to any one of claims 10-15 in preparing a polypeptide.

17. The use according to claim 16, wherein the polypeptide is present in the form of a low-toxic single-chain polypeptide in a cell or lysis buffer and has toxicity after a downstream protease activation step; preferably, the polypeptide is a gene recombinant polypeptide.

18. The use according to claim 17, wherein the polypeptide is a neurotoxin precursor and has neurotoxicity after being activated by a protease.

19. The use according to any one of claims 16-18, wherein the polypeptide comprises at least two functional amino acid structural regions, wherein a first functional amino acid structural region comprises a metal ion-dependent protease activity domain, a second functional amino acid structural region comprises a receptor-binding domain that can bind to a surface receptor of a target cell and/or a translocation domain that can mediate a transfer of the polypeptide across a vesicle membrane, and the two functional amino acid structural regions are connected via a structural region comprising a protease cleavage site.

20. The use according to claim 19, wherein the protease cleavage site is a 3C enzyme cleavage site.
